# EUROPEAN PATENT APPLICATION

(11) **EP 4 424 695 A1**
(43) Date of publication of application: **04.09.2024**
(21) Application number: 22885946.8
(22) Date of filing: 25.10.2022
(51) Int. Cl.: C07H 17/06, A61K 31/35, A61P 25/00

(54) **USE OF ISORHOIFOLIN AND DERIVATIVE THEREOF IN PROMOTING NERVE REPAIR**

(30) Priority: 25.10.2021 US 202163271411 P
(71) Applicant: Chen, Linyi, Hsinchu, Taiwan 30013 (CN); Chen, Yu-Jen, Hsinchu, Taiwan 30013 (TW)
(72) Inventor: LIAO, Wen-Ling, Hsinchu, Taiwan 30013 (TW); WANG, Yi, Hsinchu, Taiwan 30013 (TW); LEE, Yu-Tang, Hsinchu, Taiwan 30013 (TW); LU, Ting-Hsuan, Hsinchu, Taiwan 30013 (TW); LI, Chia-Wei, Hsinchu, Taiwan 30013 (TW); CHEN, Fang-Yi, Hsinchu, Taiwan 30013 (TW); CHIAO, Chuan-Chin, Hsinchu, Taiwan 30013 (TW); CHEN, Linyi, Hsinchu, Taiwan 30013 (TW); CHEN, Yu-Jen, Hsinchu, Taiwan 30013 (TW)
(74) Representative: karo IP
(86) International application number: PCT/CN2022/127372
(87) International publication number: WO 2023/072069

(57) **Abstract**

The invention relates to a new use of Isorhoifolin and its derivatives. The invention provides uses of the Isorhoifolin derivatives for promoting regeneration of injured brain neurons and retinal neurons.

## Description

### FIELD OF THE INVENTION

This disclosure relates to a use of an Isorhoifolin or its derivative to promote neural repair.

### BACKGROUND OF THE INVENTION

Current nerve injury, such as Traumatic Brain Injury (TBI), affect approximately seventy million people globally each year. Common treatment modalities include physical therapy, hyperbaric oxygen therapy, transcranial magnetic stimulation, and transcranial direct current stimulation. These non-invasive therapies can improve depression and cognitive function post-TBI, yet there is currently no effective medication to promote neural regeneration following brain injury.

Traumatic Brain Injury (TBI) is damage to the brain caused by external force or impact. There are approximately nearly 70 million confirmed cases globally each year. TBI can impair brain nerves, leading to deficits in motor or cognitive functions in patients. Due to the difficulty of central nervous system regeneration and recovery after damage, there are currently no effective therapies in medicine to promote neural regeneration for TBI. Patients with traumatic brain injuries often develop brain lesions over time, which may lead to degenerative neurological diseases in the future. Therefore, early administration of medications that promote neural regeneration following brain injury is the solution for treatment.

Furthermore, in terms of selecting therapeutic drugs, due to the presence of the blood-brain barrier, conventional medications cannot easily penetrate the blood-brain barrier to reach effective concentrations. Therefore, when treating brain disorders, the administration of effective medications becomes a significant challenge.

Given this, there is an urgent need to develop a medication for treating nerve injury. Additionally, there is a critical need for a drug delivery method capable of penetrating the blood-brain barrier.

Isorhoifolin is a known compound, albeit not extensively studied for therapeutic purposes. While formulations containing this compound have been observed for treating venous issues or hemorrhoids, its use in treating neuro-related conditions is not documented. Therefore, this invention further explores that Isorhoifolin and its derivatives can effectively promote neural repair and regeneration. Moreover, they can penetrate the blood-brain barrier, suggesting potential for developing drugs to treat traumatic brain injuries.

### SUMMARY OF THE INVENTION

The human nervous system comprises the central nervous system (CNS) and the peripheral nervous system (PNS), both composed of neurons. The trigeminal nerve is a cranial nerve within the peripheral nervous system that connects to the brainstem (central nervous system).

Therefore, when the Isorhoifolin compound and its derivatives, as described in this invention, traverse the nasal mucosa, pass through the olfactory epithelial cells, and enter the pathway surrounding the olfactory and trigeminal nerves, they can reach the brain. Simultaneously, they can also reach the peripheral nervous system, thereby achieving a systemic effect in neural repair.

Given this, in present invention, the experiment focuses on using the less reparable cranial nerves. This approach aims to achieve a reparative effect on both the central nervous system and the peripheral nervous system within the neural system.

The "central nervous system" comprises the brain and spinal cord. In the context of this invention, the term "central nervous system" includes, but is not limited to, the olfactory bulb, amygdala, hippocampus, neocortex, lateral ventricles, superior colliculus, thalamus, hypothalamus, pituitary gland, pineal gland, third ventricle, midbrain tectum, cerebral peduncle, frontal lobe, cerebral aqueduct, brainstem, cerebellum, and spinal cord.

The "peripheral nervous system" is composed of the somatic nervous system and the autonomic nervous system. In the context of this invention, the term "peripheral nervous system" includes, but is not limited to, sensory nerves, motor nerves, cranial nerves, spinal nerves, sympathetic nerves, parasympathetic nerves, and the enteric nervous system.

In view of the above-mentioned problem, the present invention provides an Isorhoifolin and its derivative for use in a method for promoting neural repair.

In some embodiments, the Isorhoifolin derivative is Narirutin.

In some embodiments, the chemical structure of the Isorhoifolin shows as structure (1) below:

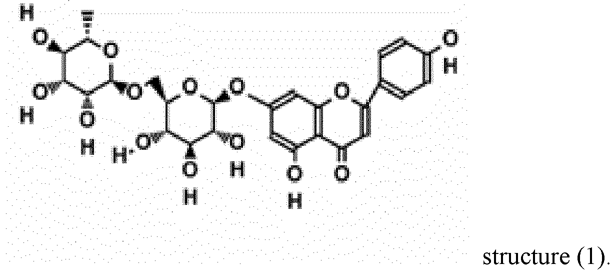

In some embodiments, the chemical structure of the Narirutin shows as structure (2) below:

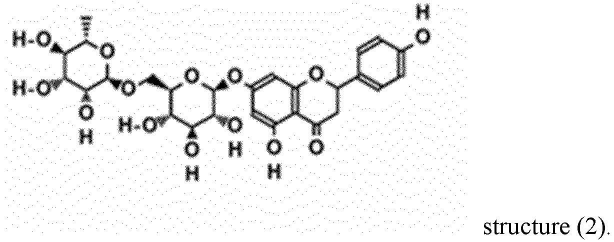

In some embodiments, the Isorhoifolin and its derivatives, when used alone, can promote the regeneration of injured hippocampal neurons by 10-30% and facilitate neural regeneration in 3D brain tissue slices. This demonstrates the effectiveness of the compound in promoting neuronal regeneration and its potential use in repairing nerve injury.

In some embodiments, the Isorhoifolin and its derivatives can penetrate the blood-brain barrier of the user, thereby entering the brain to facilitate the repair of neurons injury.

In some embodiments, the Isorhoifolin and its derivatives can penetrate the blood-brain barrier, enabling them to enter the brain and promote the repair, regeneration, or increase in the number of brain neurons.

In some embodiments, the Isorhoifolin and its derivatives can promote the regeneration of injured cortical neurons, hippocampal neurons, and retinal neurons.

In some embodiments, the Isorhoifolin exhibits low toxicity towards Neuro2a cells.

In some embodiments, the effective dose concentration of Isorhoifolin and its derivatives ranges from 1 nM to 864 µM.

In some embodiments, the Isorhoifolin and its derivatives have a significant effect on neural repair. When administered nasally, these compounds can penetrate the blood-brain barrier, facilitating the repair of damaged neurons. They promote neural repair, regeneration, or an increase in the number of neurons in the brain. Additionally, they facilitate nerve axon regeneration in injured cortical neurons and hippocampal neurons.

It is to be understood that both the foregoing general description and the following detailed description are exemplary and explanatory only and are not restrictive of the invention.

### BRIEF DESCRIPTION OF THE DRAWINGS

**Fig. 1** shows that the flowchart of *in vitro* neural repair assay in present invention.
**Fig. 2** shows that the reference schematic diagram for calculating gap closure rate in the present invention.
**Figs. 3A-3C** show that the results of the *in vitro* hippocampal neuronal repair experiments involving Isorhoifolin in the present invention.
**Fig. 4** shows that the results of the *in vitro* hippocampal neuronal regeneration experiments involving the Narirutin in the present invention.
**Fig. 5** shows that the results of the *in vitro* cortical neuronal regeneration experiments involving the Isorhoifolin derivative Narirutin in the present invention.
**Fig. 6** shows that the flowchart of *ex vivo* brain tissue slice experiments in the present invention.
**Fig. 7** shows that the results of *ex vivo* brain tissue slice experiments involving Isorhoifolin in the present invention.
Fig. 8 shows that the results of *ex vivo* brain tissue slice experiments involving the Narirutin in the present invention.
Fig. 9 shows that the results of cytotoxicity experiments involving Isorhoifolin compounds in the present invention.
Fig. 10 shows that the experimental flowchart for the Controlled Cortical Impact (CCI) model in the present invention, and the results of experiments demonstrating the promotion of motor coordination recovery in mice following brain injury by Isorhoifolin compounds.
Fig. 11 shows that the flowchart for promoting *ex vivo* retinal nerve tissue repair in the present invention.
Fig. 12 shows that the results of experiments demonstrating the promotion of *ex vivo* retinal nerve tissue repair by Isorhoifolin in the present invention.

### DETAILED DESCRIPTION OF THE INVENTION

Additional specific embodiments of the present invention include, but are not limited to the following:

### EXAMPLE 1

### In vitro neural repair assay of Isorhoifolin and its derivatives

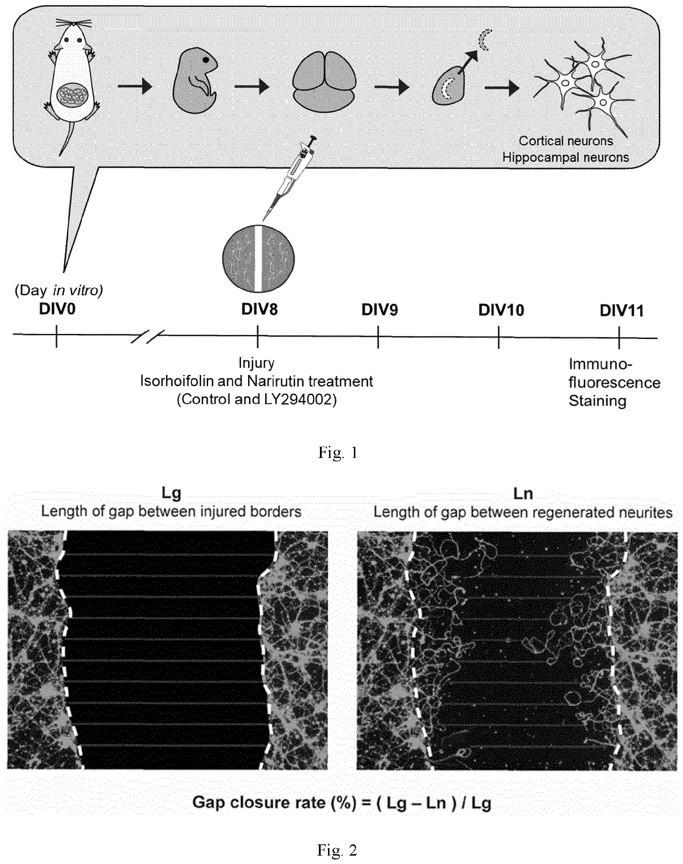

The experimental procedure is as depicted in Figure 1. Removed the brain of E18 rat embryos and dissected into cortex and hippocampus. Subsequently, the cortex and hippocampus were separated into cortical neurons and hippocampal neurons, respectively. Then, seeded hippocampal neurons on 48-well plates. The first day that neurons are cultured *in vitro* is defined as day *in vitro* 0 (DIVO). On DIV2, Cytosine beta-D-arabinoside (AraC) was added to neurons to inhibit the proliferation of glial cells. On DIV8, neurons were injured using pipette tips (p10 tips) and various concentrations of Isorhoifolin was added. After 72 hours, immunostaining was performed. TUJ1 antibody was used to label neuronal cells to observe the regrowth of neurites. Images were taken using Observer Z1 microscope.

As depicted in Figure 2, the degree of neuronal axon regeneration was quantified using the gap closure rate. White dashed lines indicate the borders of injury gap, the central black area represents the region scratched by the tip of a pipette tips. The newly regenerated neurites grow from the white dashed lines toward the center of the gap. To quantify the results, we measured the lengths of gap (Lg) and distance between regenerated neurites (Ln) every 50 µm. After unbiasely drawing ten lines of equal distance, the average distance between neurites is taken: Length of gap between regenerated neurons (Ln). Gap closure rate is calculated using the formula: (Lg-Ln)/Lg. Scale bar = 100 µm. Images were taken using Observer Z1 microscope.

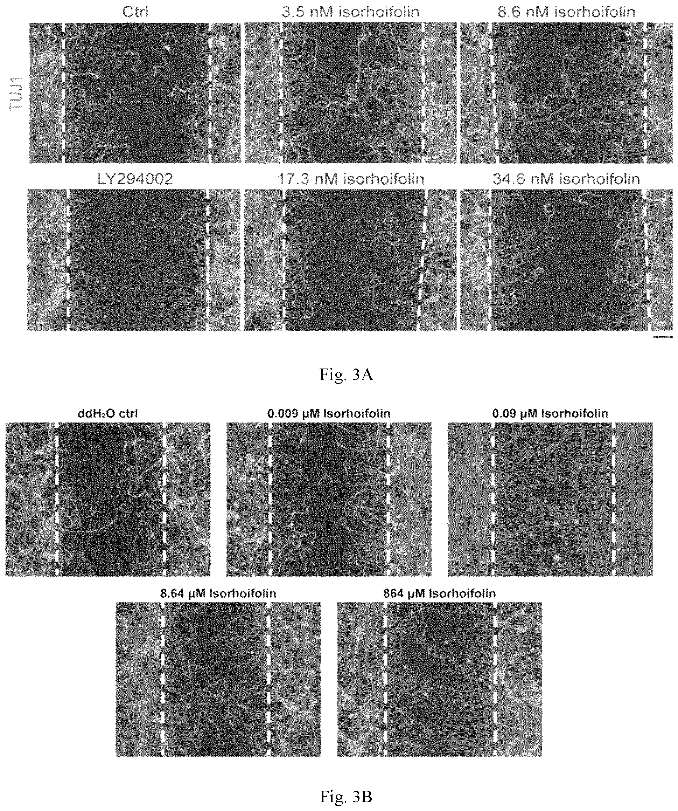

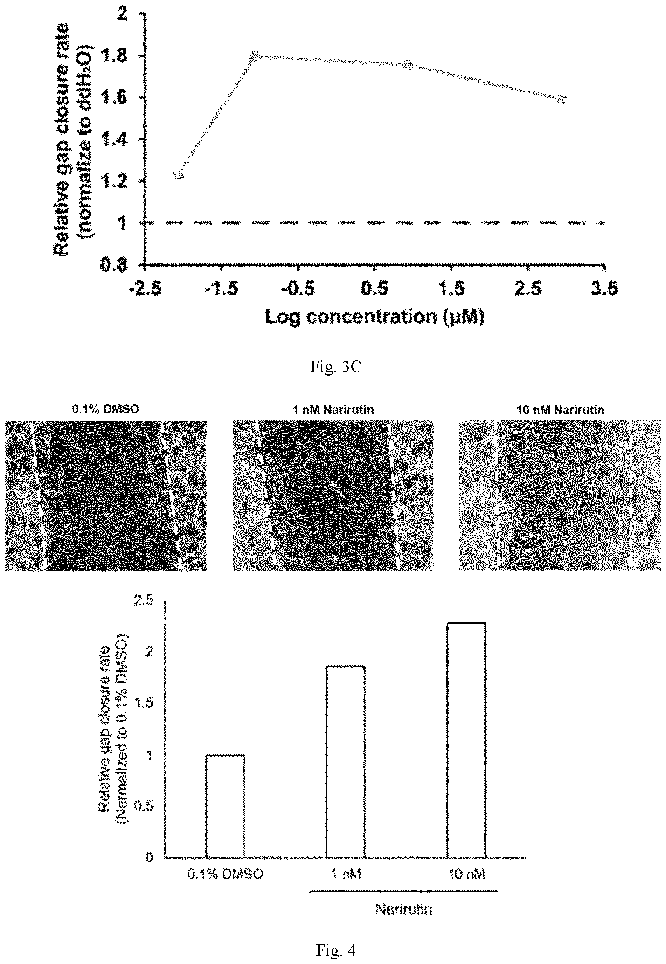

As shown in Figures 3A-3C, the white dashed line represents the boundary generated by scratching the neuronal cells with the tip of a micropipette. Scale bar = 100 µm. Images were taken using Observer Z1 microscope. This result indicates that Isorhoifolin promotes neurite regrowth after injury in hippocampal neurons.

### Experiment on promoting regeneration of injured hippocampal neuronal by the Isorhoifolin derivative Narirutin

The experimental procedure is also as depicted in Figure 1. Removed the brain of E18 rat embryos and dissected into cortex and hippocampus. Subsequently, the cortex and hippocampus were separated into cortical neurons and hippocampal neurons, respectively. Then, seeded hippocampal neurons on 48-well plates. The first day that neurons are cultured *in vitro* is defined as day *in vitro* 0 (DIVO). On DIV2, Cytosine beta-D-arabinoside (AraC) was added to neurons to inhibit the proliferation of glial cells. On DIV8, neurons were injured using pipette tips (p10 tips) and 1 nM and 10 nM Narirutin was added. 0.1% DMSO served as the solvent control group, and the results in the figures were standardized against the 0.1% DMSO group. After 72 hours, immunostaining was performed. TUJ1 antibody was used to label neuronal cells to observe the regrowth of neurites. Scale bar = 100 µm. Images were taken using Observer Z1 microscope.

As shown in Figure 4, the degree of neuronal axon regeneration was quantified using the gap closure rate. The results of this experiment indicate that Narirutin effectively promotes dendritic outgrowth in hippocampal neurons.

### Experiment on promoting regeneration of injured cortical neuronal by the Isorhoifolin derivative Narirutin

The experimental procedure is also as depicted in Figure 1. Removed the brain of E18 rat embryos and dissected into cortex and hippocampus. Subsequently, the cortex and hippocampus were separated into cortical neurons and hippocampal neurons, respectively. Then, seeded cortical neurons on 48-well plates. The first day that neurons are cultured *in vitro* is defined as day *in vitro* 0 (DIVO). On DIV2, Cytosine beta-D-arabinoside (AraC) was added to neurons to inhibit the proliferation of glial cells. On DIV8, neurons were injured using pipette tips (p10 tips) and 10 nM and 100 nM Narirutin was added. 0.1% DMSO served as the solvent control group, and the results in the figures were standardized against the 0.1% DMSO group. After 72 hours, immunostaining was performed. TUJ1 antibody was used to label neuronal cells to observe the regrowth of neurites. Scale bar = 100 µm. Images were taken using Observer Z1 microscope.

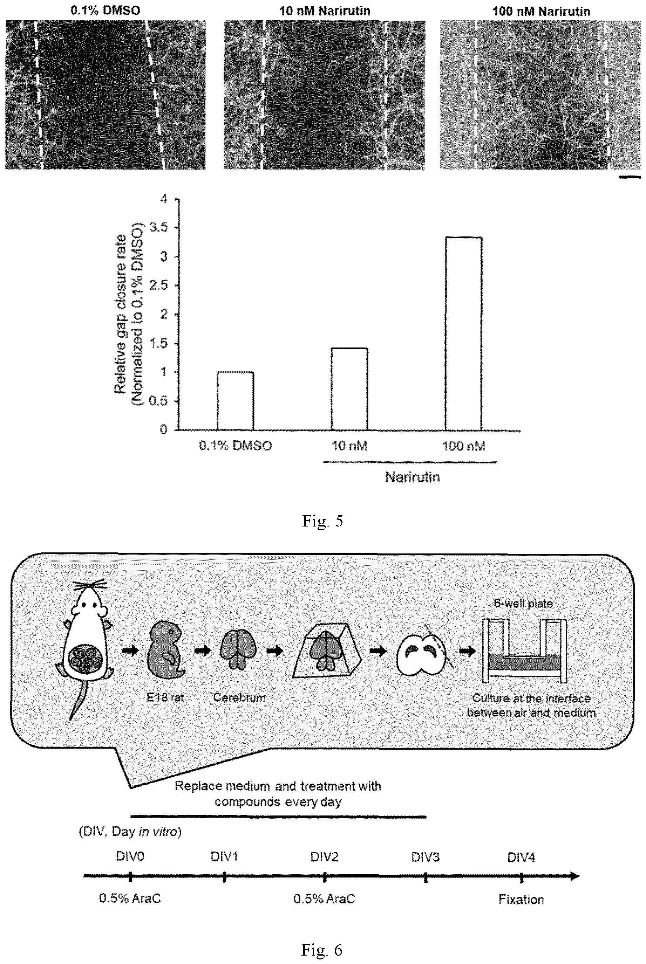

As shown in Figure 5, the degree of neuronal axon regeneration was quantified using the gap closure rate. The results of this experiment indicate that Narirutin effectively promotes dendritic outgrowth in cortical neurons.

### EXAMPLE 2

### Ex vivo brain tissue slice experiments with Isorhoifolin and its derivatives

After testing the effects of compounds on neuronal regeneration *in vitro,* we also attempted to observe the effects of the compounds on neuronal regeneration in an *ex vivo* setting.

As shown in Figure 6, the present invention utilized a method of culturing 3D brain tissue slices to observe the effects of compounds on neurons. For the experiment, we took out the brain of E18 rat embryos and embedded in low-melting agarose. The brains were sectioned using Leica microtome VT100. The tissue sections were collected with 350 µm thick and injured using a scalpel. And placed them in inserts within a 6-well plate. This culturing method allows the brain slices to be exposed to both medium and air, facilitating a more realistic representation of the interactions between neuronal cells and other brain cells, as well as the three-dimensional structure, compared to *in vitro* culture. ddH₂O or Isorhoifolin was added to injured brain sections every day. After 96 hours, immunostaining was performed. TUJ1 antibody was used to label neuron cells. GFAP antibody was used to label glial cells. DAPI was used to label nucleus.

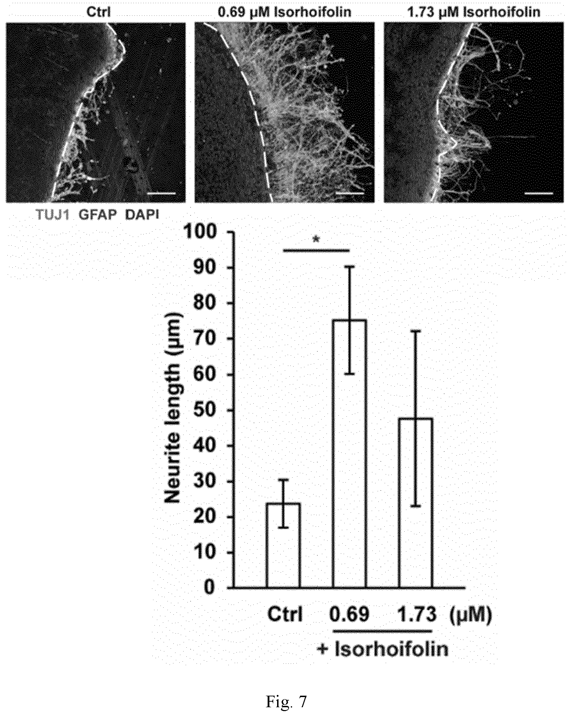

As shown in Figure 7, the white dashed line indicated the injury site caused by a scalpel. The regenerated neurites grow from injury site toward the right side of the images. Scale bar = 100 µm. Images were taken using Zeiss LSM800 confocal microscope. The result shows that Isorhoifolin promotes neurite outgrowth after injury in *ex vivo* 3D brain slice culture.

The experimental procedure for the Isorhoifolin derivative Narirutin is also depicted in Figure 6. Took out the brain of E18 rat embryos and embedded in low-melting agarose. The brains were sectioned using Leica microtome VT100. The tissue sections were collected with 350 µm thick and injured using a scalpel. And placed them in inserts within a 6-well plate. This culturing method allows the brain slices to be exposed to both medium and air, facilitating a more realistic representation of the interactions between neuronal cells and other brain cells, as well as the three-dimensional structure, compared to *in vitro* culture. ddH₂O or Narirutin was added to injured brain sections every day. After 96 hours, immunostaining was performed. TUJ1 antibody was used to label neuron cells. GFAP antibody was used to label glial cells. DAPI was used to label nucleus. Images were taken using Zeiss LSM800 confocal microscope.

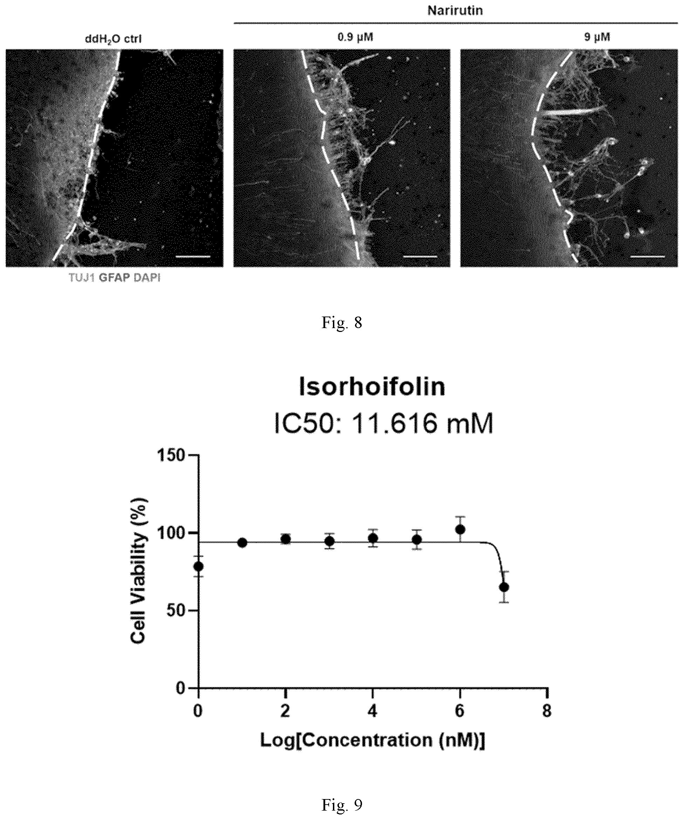

As shown in Figure 8, the result shows that Narirutin promotes neurite outgrowth after injury in *ex vivo* 3D brain slice culture.

### EXAMPLE 3

### Isorhoifolin cytotoxicity experiment

Utilizing the CellTiter-Glo cell viability assay to assess the toxicity of Isorhoifolin on Neuro2a cells. CellTiter-Glo cell viability assay can detect the level of ATP in neuro2a cells after treatment of compounds to evaluate the survival rate.

As shown in Figure 9, the results shows that the IC50 of Isorhoifolin is about 11.616 mM, which suggest a good safety profiles of these compounds.

### EXAMPLE 4

### Experiment on the Promotion of Motor Coordination Recovery in Mice Following Brain Injury by Isorhoifolin

Using the horizontal bar test, mice were placed on brass rods positioned at a height of 49 cm above the ground, with lengths of 38 cm and diameters of 2 mm, 4 mm, and 6 mm. Mice were required to grasp the brass rod with their forepaws, and their ability to move and coordinate was assessed by measuring the time spent on the brass rod and whether they reached the platform at the end of the rod. Evaluation criteria were as follows: 1 point for 1-5 seconds, 2 points for 5-10 seconds, 3 points for 10-20 seconds, 4 points for 20-30 seconds, and 5 points for over 30 seconds or reaching the platform.

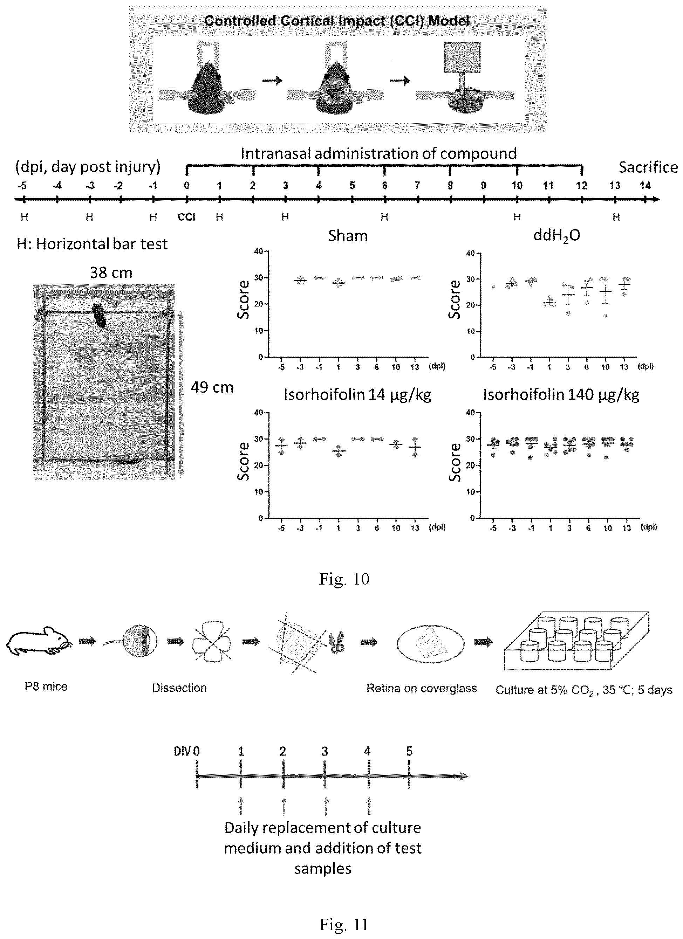

The experimental model, as depicted in Figure 10, involved pre-training at -5, -3, and - 1 days post injury (Dpi) (5, 3, and 1 day(s) before brain injury). At 0 Dpi, the controlled cortical impact model (CCI model) was used to induce brain injury in mice. Subsequently, at 0, 2, 4, 6, 8, 10, and 12 Dpi, mice were administered Isorhoifolin at doses of 14 or 140 µg/kg via intranasal delivery. Horizontal bar experiments were conducted at 1, 3, 6, 10, and 13 Dpi.

As shown in Figure 10, it is evident that the group receiving only water (ddH₂O) after brain injury exhibited a loss of motor coordination. However, in the groups receiving Isorhoifolin, motor coordination was similar to that of the Sham (surgery control) group, indicating a significant promotion of motor coordination recovery.

### EXAMPLE 5

### Isorhoifolin promotes ex vivo retinal neuronal tissue repair

The experimental design and procedure are showed in Figure 11. Retinal explants were obtained from C57BL/6 mice at postnatal day 8. Following the sacrifice of mice, the eyes were enucleated, and the eyeballs were dissected using forceps and microscissors to separate the retina and remove the vitreous. Subsequently, the dissected retinas were quartered and trimmed along the edges of the tissue using microscissors. Each quarter was cultured on an 18 mm round coverslip and placed in a 12-well plate inside a CO₂ incubator at 35°C for five days. The culture medium was replaced daily with fresh medium containing either 9 µM or 90 µM Isorhoifolin. On the fifth day of culture, the retinal tissues were fixed with a mixture of 0.1% glutaraldehyde solution and 4% paraformaldehyde at room temperature for one hour. Immunostaining was performed using primary antibodies against the axonal marker beta-III-tubulin (TUJ1) and DAPI to label neurons and cell nuclei, respectively. Images were acquired using a super-resolution upright confocal microscope (LSM-800, Carl Zeiss). Imaged software was utilized for image analysis, wherein the tissue boundary was delineated to calculate the perimeter and the area of neuronal fibers outside the tissue boundary. The ratio of neuronal fiber area to tissue boundary perimeter was then calculated to determine the unit perimeter neuronal fiber length.

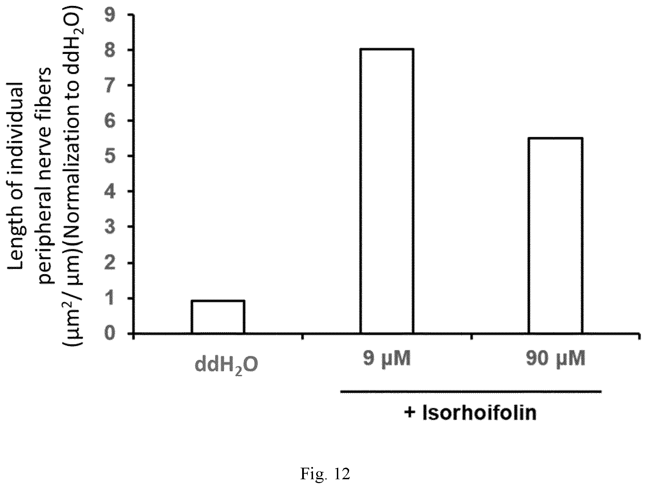

As shown in Figure 12, the group treated with Isorhoifolin exhibited significant recovery of injured retinal neurons compared to the group treated with water (ddH₂O) alone.

All examples provided herein are intended for pedagogical purposes of aiding the reader in understanding the invention and the concepts contributed by the inventors to further the art, and are not to be construed as limitations to such specifically recited examples and conditions, nor does the organization of such examples in the specification relate to a showing of the superiority or inferiority of the invention. Although one or more embodiments of the present invention have been described in detail, it should be understood that the various changes, substitutions, and alterations could be made hereto without departing from the spirit and scope of the invention.

It is intended that the specification and examples be considered as examples only, with a true scope and spirit of the invention being indicated by the following claims.

## Claims

1. A method for preparing a dug treating nerve injury, wherein the drug is Isorhoifolin and an Isorhoifolin derivative.

2. The method of claim 1, wherein the Isorhoifolin derivative is Narirutin.

3. The method of claim 1, wherein the neural injury is central nervous system or peripheral nervous system injury.

4. The method of claim 1, wherein the nerve injury is a neuron injury.

5. The method of claim 1, wherein the nerve injury comprises cortical neurons, hippocampal nerves, or retinal neurons.

6. The method of claim 1, wherein the treating is neural regeneration, increase in the number of neurons, or neural repair.

7. The method of claim 1, wherein the Isorhoifolin and the Isorhoifolin derivative, when administered nasally, can penetrate the blood-brain barrier to enter the brain.

8. The method of claim 1, wherein the effective dose range for the Isorhoifolin and the Isorhoifolin derivatives is from 1 nM to 864 µM.
